# EUROPEAN PATENT SPECIFICATION

(11) **EP 0 496 832 B1**
(45) Date of publication and mention of the grant of the patent: **11.12.1996**
(21) Application number: 90917479.9
(22) Date of filing: 22.10.1990
(51) Int. Cl.: C12N 5/06, A61K 38/00

(54) **INHIBITION OF PADGEM-MEDIATED CELL BINDING**
HEMMUNG VON PADGEM-VERMITTELTER ZELLBINDUNG
INHIBITION DE LA LIAISON DES CELLULES A MEDIATION PAR PADGEM

(30) Priority: 20.10.1989 US 424886
(43) Date of publication of application: 05.08.1992
(73) Proprietor: NEW ENGLAND MEDICAL CENTER, Boston, MA 02111 (US)
(72) Inventor: FURIE, Bruce, Wellesley, MA 02181 (US); LARSEN, Eric, Roslindale, MA 02131 (US); CELI, Alessandro, Boston, MA 02199 (US); GILBERT, Gary, E., Melrose, MA 02176 (US); FURIE, Barbara, C., Wellesley, MA 02181 (US); ERBAN, John, K., Wakefield, MA 01880 (US); BONFANTI, Roberta, Boston, MA 02199 (US); WAGNER, Denisa, D., Wellesley, MA 02181 (US)
(74) Representative: Deans, Michael John Percy
(86) International application number: US9006101
(87) International publication number: WO9106632

(56) References cited:
- EP-A- 0 317 278
- WO-A-92/01718
- Cell, Vol. 56, issued 24 March 1989, JOHNSTON, et al."Cloning of GMP-140, a Granule Membrane Protein of Platelets and Endothelium: Sequence Similarity to Proteins Involved in Cell Adhesion and Inflammation". See entire document, pages 1033-44.
- Blood Vol. 67, No. 3 issued March 1986, JUNGI, et al. see entire document, pages 629-636.
- Science, Vol. 243, issued 03 March 1989, SIEGELMAN, et al., "Mouse Lymph Node Homing Receptor cDNA Clone Encodes a Glycoprotein Revealing Tandem Interaction Domains", see page 1171.
- Science, Vol. 243, issued 03 March 1989 BEVILACQUA ET AL, "Endothelial Leukocyte Adhesion Molecule 1: An Inducible Receptor for Neutrophils Related to Complement Regulatory Proteins and Lectins", pages 1160-1165, see page 1162.

## Description

This invention relates to inhibition of PADGEM-mediated cell binding and is useful, for example, in the prevention of blood cell aggregation.

Blood platelets are anucleate cells which circulate in the blood in a resting, inactive form. During the initiation of hemostasis, these cells become activated and undergo major morphological, biochemical, and functional changes, e.g., rapid granule exocytosis, or degranulation, in which the platelet alpha granule membrane becomes fused with the external plasma membrane and new cell surface proteins become expressed that confer on the activated platelet new functions, e.g., the ability to bind both other activated platelets and other cells. Activated platelets are recruited into growing thrombi or are cleared rapidly from the blood circulation. Activated platelets bind to phagocytic white cells, including monocytes and neutrophils (Jungi et al. (1986), Blood 67, 629-636), and also to monocyte-like cell lines, e.g., HL60 and U937 (Jungi et al., 1986, supra; Silverstein et al. (1987), J. Clin. Invest. 79, 867-874).

PADGEM (platelet activation dependent granule-external membrane protein), also known as GMP-140, is an alpha granule membrane protein of molecular weight 140,000 that is expressed on the surface of activated platelets upon platelet stimulation and granule secretion (Hsu-Lin et al. (1984), J. Biol. Chem. 259, 9121-9126; Stenberg et al. (1985), J. Cell Biol. 101:880-886; Berman et al (1986), J. Clin. Invest. 78, 130-137). It is also found in megakaryocytes (Beckstead et al., 1986, Blood 67, 285-293), and in endothelial cells (McEver et al 1987), Blood 70:3558)) within the Weibel-Palade bodies (Bonfanti et al (1989), Blood 73), 1109-1112. Furie et al US Patent No: 4 783 330, describes monoclonal antibodies reactive with PADGEM.

We have discovered that PADGEM mediates the binding of cells which bear PADGEM, e.g., activated platelets and stimulated endothelial cells, to cells, in particular leukocytes such as monocytes and neutrophils, which bear a PADGEM-specific ligand. Our discovery permits the inhibition of binding of PADGEM-bearing cells to PADGEM-specific ligand-bearing cells, by use of a substance which binds either to PADGEM or to a PADGEM-specific ligand. Examples of biological processes which are or can be pathological, and which can be inhibited following the teaching set out herein are atherosclerosis, clotting, and inflammation.

Our models for atherosclerosis, and prophylactic anti-atherosclerotic therapy, are as follows. An injured endothelial cell in a vessel wall expresses PADGEM on its surface. This recruits PADGEM ligand-bearing monocytes to the region, and those monocytes, mediated by PADGEM-ligand binding, adhere to the endothelial cells, and eventually become pathologic foam cells by ingestion of lipids, platelet fragments, and other molecules. The cycle is interrupted by administration of a soluble agent which competitively inhibits the binding of endothelial cells to monocytes by either binding to PADGEM on the endothelial cells or PADGEM-specific ligand on the monocytes. If activated platelets are also involved in the recruitment of monocytes by virtue of their bearing PADGEM, that recruitment will be inhibited as well. In addition, if, in addition to monocytes, PADGEM-bearing cells bring about recruitment of neutrophils, which secrete deleterious enzymes, that process will also be inhibited.

Our model for clotting is as follows. At the beginning of the coagulation process, activated platelets accumulate on injured surfaces of the vessel walls. The activated platelets, because they express PADGEM, cause recruitment of monocytes to the area. The monocytes secrete or otherwise cause the accumulation in the region of Tissue Factor, a protein which initiates the coagulation cascade. Our inhibitory substances described hereinbelow interrupt the process by preventing binding of monocytes to the activated platelets.

Our model for inflammation is as follows. Activated platelets accumulate at the site of tissue injury and, by virtue of PADGEM on their surfaces, recruit monocytes and neutrophils to the area. Those monocytes then deliver inflammatory components to the site of injury. Inhibition prevents monocyte-platelet binding and neutrophil-platelet binding.

Thus, the invention provides, in a first aspect thereof a method of inhibiting, in vitro, the binding of a first cell bearing PADGEM to a second cell bearing a PADGEM-specific ligand, comprising contacting said sample with an inhibiting substance which binds either to PADGEM or to said PADGEM-specific ligand to inhibit the binding of said first cell to said second cell.

In a second and alternative aspect thereof, the invention provides a soluble protein fragment capable of mimicking a site of PADGEM which binds to a PADGEM-specific ligand on a leukocyte.

The invention also extends to use of compositions comprising an inhibiting substance which binds to PADGEM for the manufacture of medicaments.

The inhibiting substance can either be one which mimics a ligand binding site on PADGEM, and thus binds to leukocytes to prevent the deleterious cell binding event; or can be a carbohydrate which mimics the binding activity of the ligand on the leukocyte and thus binds to PADGEM to inhibit the deleterious cell-cell binding event. Where the substance mimics the ligand binding site of PADGEM, it is preferably a soluble protein fragment which excludes the hydrophobic transmembrane region of PADGEM, but includes a sufficient amount of PADGEM to cause the fragment to bind to the ligand on leukocytes. The soluble PADGEM fragment preferably contains at least 4, and more preferably 10 to 15 amino acids, and is at least 90% homologous with a region of the naturally-occurring PADGEM molecule. The fragment, in addition to excluding the transmembrane region, can include, for example, the lectin domain, but not other regions of the PADGEM molecule; the lectin domain, as described in McEver, infra, is the region defined by amino acids 1-118. Another candidate molecule is one of the C3b repeating domains of PADGEM. Either the lectin domain by itself, the C3b-C4b regulatory protein repeat domain by itself, or another domain, can be sufficient to effect the desired competitive inhibition, which requires only binding, but not biological activity, of the fragment.

Other features and advantages of the invention will be apparent from the following description of preferred embodiments thereof.

We first briefly describe the drawings.

Fig. 1 is a photograph showing the effects of anti-PADGEM antibodies and purified PADGEM on the interaction of platelets with HL60 cells and neutrophils

Fig. 2 is a graph quantitating the effect of different agents on the interaction of HL60 cell adherence to activated platelets.

Figs. 3(a) and 3(b) are graphs demonstrating quantitative inhibition of the adherence of activated platelets to U937 cells by PADGEM and anti-PADGEM antibodies.

Fig. 4 is a graph showing interaction of resting and activated platelets with various cell types

Fig. 5 is a photograph showing binding of phospholipid vesicles containing PADGEM to neutrophils and U937 cells.

Fig. 6 is a graph presenting quantitative analysis of binding of PADGEM-containing phospolipid vesicles to U937 cells.

Fig. 7 is a schematic illustration of a composite nucleotide sequence encoding PADGEM and the deduced amino acid sequence (adapted from Johnston et al. (1989), Cell, 56:1033).

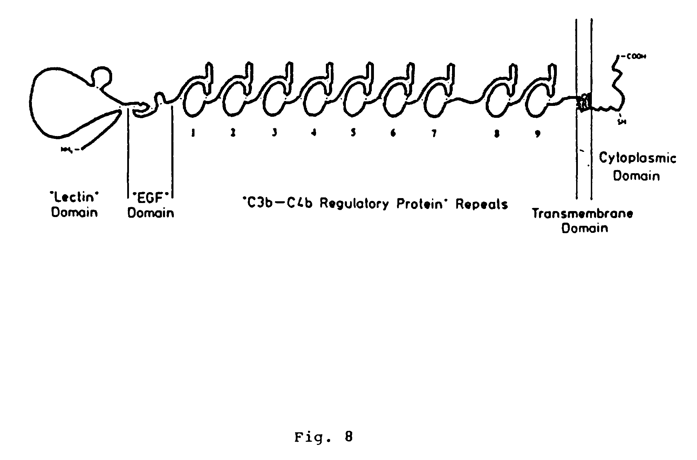

Fig. 8 is a diagrammatic illustration of the PADGEM protein, indicating protein domains.

Figs. 9-10 are graphs illustrating the platelet/HL-60 binding inhibition of fucoidin, chondroitin sulfate, and heparin. (All three are sulfated carbohydrates.)

### The PADGEM Protein

PADGEM is a receptor protein that mediates the binding of activated platelets to neutrophils and monocytes, as described below and stimulated endothelial cells to neutrophils and monocytes. The DNA sequence of PADGEM (Johnston et al., id) indicates a domain structure including a lectin domain, an epidermal growth factor-like domain, concensus repeat units, a transmembrane domain, and a short cytoplasmic domain. The predicted primary structure of PADGEM shows structural homology with ELAM-1 and MEL-14, two proteins involved in vascular cell-cell interaction (Bevilacqua et al. (1989), Science 243, 1160-1165; Seigelman et al. (1989), Science 243, 1165-1172, Laskey et al. (1989), Cell 56, 1045-1055).

The experiments described below demonstrate that PADGEM can function as a heterotypic intercellular adhesion molecule. These experiments show that activated platelets but not resting platelets bind to cells that bear a PADGEM receptor, e.g., HL60 cells, U937 cells, monocytes, and neutrophils; that PADGEM mediates this binding interaction since anti-PADGEM antibody specifically inhibits binding; that neither resting or activated platelets interact with other types of vascular cells, i.e., those that do not bear the PADGEM receptor; and that phospholipid vesicles containing PADGEM specifically bind cells that bear the Padgem receptor.

Various protein fragments of PADGEM may be used to treat thrombosis and inflammation by competitive inhibition of PADGEM-mediated adherence of blood cells. PADGEM protein fragments derived from proteolytic digestion of the natural molecule or synthetic PADGEM protein fragments may be screened for their ability to inhibit platelet-HL60 binding, as described below. A desired PADGEM protein fragment may be produced to inhibit PADGEM-expressing cells from adhering to PADGEM ligand-expressing cells either from expression of synthetic DNA encoding the fragment or from expression of a cloned fragment of the natural PADGEM gene, also as described below.

### Effects of PADGEM and Anti-PADGEM Antibodies on the Interaction of Platelets with HL60 Cells and Other Cells

To show that PADGEM mediates activated platelet-HL60 interaction, a cellular adhesion assay was performed in the presence of purified PADGEM protein. The specificity of the activated platelet-HL60 interaction was tested using anti-PADGEM antibodies in an attempt to saturate PADGEM receptors on monocyte-like human HL60 cells, and thus block rosette formation. PADGEM-mediated platelet binding was also tested using neutrophils and the monocyte-like human cell line U937. These results are described below.

### Isolation and Maintenance of Platelets, Neutrophils, HL60, and U937 Cells

Platelets were isolated by gel filtration from fresh anticoagulated blood obtained from normal human donors (Hsu-Lin et al., 1984, supra). Activated platelets were prepared by incubating cells without stirring for 20 min at 22° with thrombin (Sigma, St. Louis, MO) at a final concentration of 0.25 U/ml. Fresh platelets were used in cell adhesion assays within 30 min of preparation. Neutrophils were prepared by the method of English and Anderson (1974), J. Immunol. Methods 5, 249-252. The neutrophil preparations were greater than 95% pure by light microscropy.

Cell lines HL60 and U937 (A.T.C.C. Nos. CCL240 and CRL1593, respectively) were maintained in culture in RPMI 1640 medium (M.A.Bioproducts, Walkersville, MD) supplemented with penicillin G sodium (100 U/ml), streptomycin sulfate (100 µg/ml), HEPES (10mM) (0.14 M NaCl, 12mM NaHCO₃, 0.008 M KCl, 0.001 M MgCl₂, 0.45 g Hepes (N-2-Hydroxyethylpiperazine-N-2-ethanesulfonic acid, Sigma, St. Louis, MO), 1 g dextrose, pH 7.35), sodium pyruvate (1 mM), L-glutamine (2 mM), β-mercaptoethanol (0.0004%) and 10% fetal calf serum.

### Preparation of Monoclonal Anti-PADGEM Antibody AC1.2

Monoclonal antibodies directed against PADGEM were prepared using the same strategy originally employed to produce KC4 (Hsu-Lin et al. (1984), supra; and U.S. Patent No. 4,783,330). Balb/c mice (Jackson Labs, Bar Harbor, ME) were immunized with thrombin-activated platelets. Splenocytes were fused with NS1 cells (A.T.C.C. No. TIB18) using standard methods (Kohler & Milstein. 1975, Nature 256, 495-497). A hybridoma, designated AC1.2, had characteristics similar to KC4 (Hsu-Lin et al., supra). This antibody, an IgG₁, binds activated platelets but not resting platelets, reacts with purified PADGEM, reacts with a 140,000 molecular weight band after SDS gel electrophoresis and Western blotting of detergent-solubilized platelets, and localizes specifically to the Weibel-Palade bodies of fixed permeabilized unstimulated endothelial cells (Bonfanti et al., (1989), supra). AC1.2 was iodinated by the lactoperoxidase-glucose oxidase method (Enzymobead, BioRad, Richmond, CA) according to the manufacturer's protocol.

### Isolation of Padgem Protein

PADGEM was purified from platelets by immunoaffinity chromatography as previously described, with minor modifications (Hsu-Lin et al., (1984), supra). Briefly, 50 units of frozen platelets were thawed, washed and sonicated. The platelet lysate was sedimented by centrifugation at 100,000 x g for 30 min at 4°C, then the pellet sonicated in 1% Lubrol PX (Sigma) and subjected to centrifugation at 100,000 x g for 30 min at 4°C. The supernatant was applied to an AC1.2-Sepharose column (Sepharose, Pharmacia Fine Chemicals, Naperville, IL), made according to conventional techniques. After extensive washing of the column in buffer without detergent, the bound protein was eluted with diethylamine, exhaustively dialyzed, concentrated and redialyzed against Tris Buffered Saline (TBS), pH 7.5 (0.02M Tris/Cl, 0.14 M NaCl). This preparation was applied to a non-immune IgG-Sepharose column equilibrated with TBS, pH 7.5. In some preparations, the PADGEM was further purified by SDS gel electrophoresis and electroelution.

### Isolation of Polyclonal Antibodies

Polyclonal antibodies were raised in rabbits using a standard immunization schedule, and anti-PADGEM antibodies isolated by affinity chromatography on PADGEM-Sepharose (Berman et al. (1986), Blood 67, 285-293). These antibodies have been previously demonstrated to bind only to activated platelets (Berman et al., (1986), id.; Palabrica et al., (1989), Proc. Natl. Acad. Sci. U.S.A. 86, 1036-1040), and to interact solely with PADGEM upon Western blotting of detergent-solubilized platelets (Berman et al. (1986), supra).

The following antigens and their antibodies were used: thrombospondin and polyclonal rabbit anti-thrombospondin antibodies (Silverstein et al., 1987, supra), GPIIb-IIIa, rabbit anti-GPIIb-IIIa, and monoclonal and polyclonal antibodies to GPIV (Silverstein et al., J. C. I., supra) (33 µg/ml), and monoclonal antibodies to PADGEM (1-18, 2-15, 2-17) (Hsu-Lin et. al, 1984, supra).

### Cell Adhesion Assays

Twenty microliters of platelet suspension (2 x 10⁸/ml) were mixed with 20 µl of cell suspension (2 x 10⁶/ml) and incubated for 20 min at 22°C in a microfuge tube. An aliquot of the cell suspension was then placed in a Neubauer chamber and evaluated by light microscopy using an Olympus model BH-2 microscope. Three samples from each assay were evaluated by counting 200 cells and scoring the percentage of cells bound to two or more adherent platelets. Antibody inhibition studies were performed by preincubataing 20µl of platelet suspension (2 x 10⁸/ml) with 20 µl of antibody solution for 20 min at 22°C. Cells (20 µl; 3 x 10⁶ cells/ml) were added to the incubation mixture for 20 min at 22°C. In some experiments, 20 µl of cells (3 x 10⁶ cells/ml) were preincubated with 20 µl of purified PADGEM, thrombospondin or bovine serum albumin (Sigma, St. Louis, MO) for 20 min at 22°C. Subsequently, 20 µl of platelet suspension was added for 20 min at 22°C.

### Results

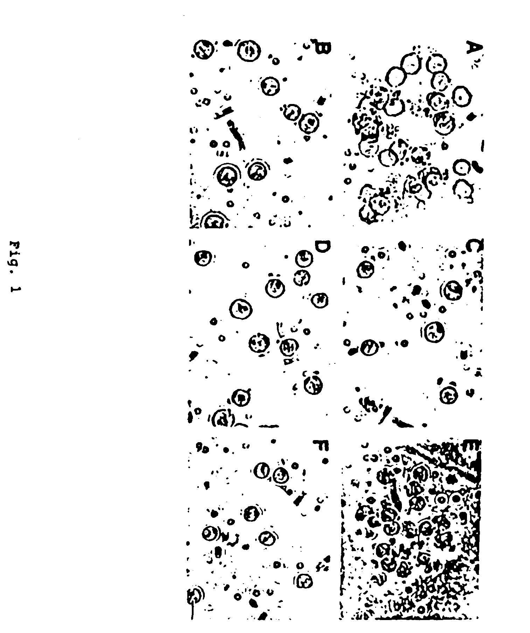

Thrombin-activated platelets, which bear PADGEM on their surface, when incubated with HL60 cells, cause HL60 cells to form rosettes, as shown in Fig. 1a. In contrast, resting unstimulated platelets, which do not express PADGEM on their surface, do not bind to HL60 cells or cause formation of rosettes (Fig. 1b). Monospecific immunoaffinity-purified polyclonal rabbit anti-PADGEM antibodies (50 µg/ml) nearly completely inhibited activated platelet-HL60 cell binding, as shown in Figs. 1c and 1f.

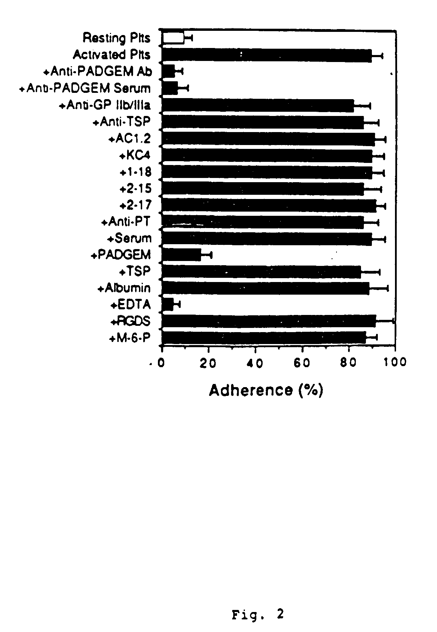

Although polyclonal anti-PADGEM antibodies inhibited cellular adhesion, five monoclonal antibodies directed against PADGEM (KC4, (42 µg/ml), AC1.2 (130 µg/ml), 1-18, 2-15, and 2-17 (all at a 1:100 dilution of ascites)) failed to inhibit platelet-HL60 cell binding, as shown in Fig. 2. Polyclonal anti-PADGEM antiserum (1:100 dilution) inhibited cellular adhesion, whereas polyclonal and monoclonal anti-thrombospondin antibodies anti-TSP (1:100 dilution), polyclonal anti-GPIIb-IIIa antibodies (anti-GP IIb/IIIa), polyclonal and monoclonal anti-GPIV antibodies (not shown), as well as anti-prothrombin antiserum (anti-PT) (1:100 dilution) and preimmune serum (serum), failed to inhibit HL60 cell-activated platelet binding (Fig. 2). In Fig. 2, resting platelets are indicated by an open bar, whereas activated platelets are indicated by a filled bar. The percentage of HL60 cells bound to two or more platelets was determined under phase microscopy.

If PADGEM is a component of a complex linking activated platelets and HL60 cells, saturation of the PADGEM recognition sites on HL60 cells with soluble PADGEM should inhibit the binding of activated platelets to these cells. As shown in Fig. 1d, purified PADGEM (30 µg/ml) incubated with HL60 cells prior to the addition of activated platelets inhibited activated platelet-HL60 cell binding by 80%. Fig. 2 shows that EDTA (5mM) also inhibited binding. In contrast, thrombospondin (TSP) (100 µg/ml), albumin (10 µg/ml), mannose-6-phosphate (M-6-P) (10 mM) and the peptide Arg-Gly-Asp-Ser (RGDS) (Peninsula Laboratories, Belmont, CA) (3mM) failed to inhibit activated platelet-HL60 binding (Fig. 2).

Neutrophils (20µl; 2 x 10⁶ cells/ml) also interacted with thrombin-activated platelets (Fig. 1e), but not with resting platelets. Antibodies to PADGEM blocked this interaction (Fig. 1f), and purified PADGEM inhibited activated platelet binding to neutrophils and to peripheral blood monocytes.

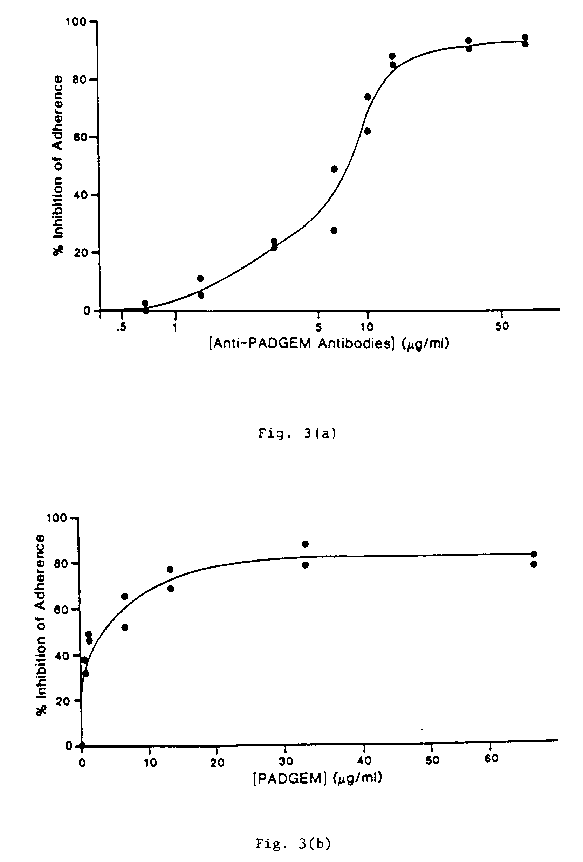

Figs. 3a and 3b show antibody-inhibition of binding of activated platelets to U937 cells by either Padegem or anti-PADGEM antibodies. The interaction of activated platelets with U937 cells was monitored by the formation of rosettes. In Fig. 3a, when affinity-purified polyclonal rabbit anti-PADGEM antibody was incubated for 20 min. with thrombin-activated platelets prior to platelet/U937 cell incubation, half-maximal inhibition occurred at 7 µg/ml of anti-PADGEM antibody and complete inhibition at antibody concentrations over 20 µg/ml. PADGEM protein also inhibited activated platelet/U937 cell adherence. Fig. 3b shows that after incubation of PADGEM protein with U937 cells (3 x 10⁶ cells/ml for 20 min. prior to incubation of U937 cells with activated platelets), half-maximal inhibition of binding occurred at 2 µg/ml of PADGEM, and maximal inhibition at PADGEM concentrations above 30 µg/ml.

The expression of PADGEM on the activated platelet has been shown to be agonist-independent (Hsu-Lin et al. (1984), supra). In addition to thrombin-stimulated platelets, ADP-, collagen-, and epinphrine-stimulated platelets bind to HL60 and U937 cells. These interactions are inhibited by PADGEM and anti-PADGEM antibodies.

### Effects of PADGEM on the Interaction of Platelets with Other Vascular Cells

Monocytes were prepared by washing the mononuclear leukocyte fraction with human serum/5 mM EDTA twice and incubating the cells in RPMI/10% fetal calf serum in sterile plastic dishes for 2 hrs at 37°C. The dishes were washed three times with Phosphate Buffered Saline (PBS) at 37°C to remove non-adherent cells. PBS at 0°C was added and the cells incubated at 4°C for 1 hr. Adherent cells were gently detached with a rubber policeman, washed in PBS, and resuspended in RPMI/1% fetal calf serum. Lymphocytes were obtained by washing the non-adherent cells with PBS and resuspending these cells in RPMI/1% fetal calf serum. The purity of these preparations was established to be greater than 90% by light microscopy using Wright, specific esterase and non-specific esterase stains. Jurkatt (A.T.C.C. No. CRL8163), CEM (A.T.C.C. No. CCL119) and Daudi (A.T.C.C. No. CCL213) cell lines were maintained in culture as described above for HL60 and U937 cells.

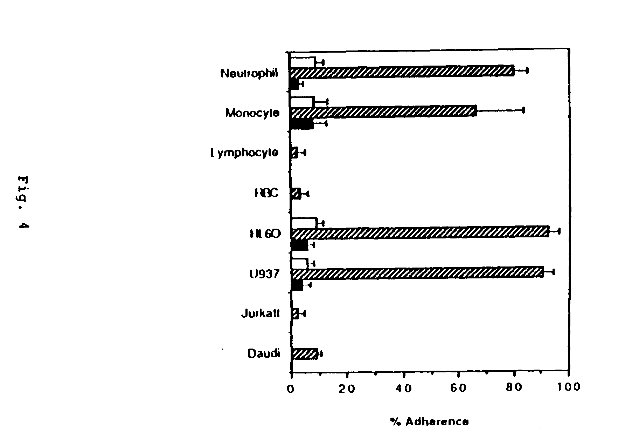

Fig. 4 shows the interaction of resting and activated platelets with various cell types. The percentage of cells observed to bind two or more platelets was determined under phase microscopy. Cells tested included neutrophils, monocytes, lymphocytes, red cells (RBC), HL60 cells, U937 cells, CEM cells, Jurkatt cells, and Daudi cells; all cells were at concentrations of 1 x 10⁶ cells/ml; platelet concentration was 1.5 x 10⁸ cells/ml. Open bars represent resting platelets alone; hatched bars represent activated platelets; and solid bars represent activated platelets incubated with anti-PADGEM antibodies. Fig. 4 shows that monocytes and neutrophils bind to activated platelets, but not normal lymphocytes, red blood cells, the human T-cell like Jurkatt cell line, or the human B-cell like Daudi cell line. Thus phagocytic cells, including monocytes and neutrophils, contain a recognition site on the cells membrane that binds to PADGEM expressed on the surface of activated platelets; this site is called the PADGEM receptor.

### Cell Binding of Phospholipid Vesicles Containing PADGEM

Since PADGEM is an integral membrane protein (Berman et al., 1986, supra) and appears to contain a transmembrane domain (Johnston et al., 1989, supra), purified PADGEM was incorporated into phospholipid vesicles composed of phosphatidylcholine and a fluorescent phosphatidylcholine analog, NBD-phosphatidylcholine (2-(6-(N-(7-nitrobenzy-2-oxa-1, 3-diazol-4-yl)amino)caproyl-3-pamitoyl-L-α-phosphatidylc holine) in order to determine if membrane (i.e., vesicle) bound PADGEM would bind to PADGEM receptor-containing cells.

PADGEM was incorporated into phospholipid vesicles using the method of Rivnay and Metzer (1982), J. Biol. Chem. 257, 12800-12808. Briefly, 5 mg of phosphatidylcholine and 0.25 mg of NBD-labeled phosphatidylcholine (Avanti Polar Lipids, Pelhams AL) in chloroform were mixed, and the chloroform removed by evaporation at 37°C. The dried lipids were resuspended in methylene chloride and the solvent removed by evaporation twice. PADGEM (1 ml; 200 µg/ml TBS) or GPIIb-IIIa (400 µg/ml TBS) was added to the dried phospholipids, CHAPS (3-[(3-cholamidopropyl) dimethylammonio]1-propanesulfonate)(Sigma) was added to a final concentration of 10 mM, and the lipids were resuspended. The preparations were dialyzed under argon against TBS/0.02% NaN₃ for two hours, the sedimented material was resuspended and dialysis continued for 24 hrs. Vesicles were separated from unincorporated protein by gel filtration on a Sepharose 4B column. The incorporation of PADGEM into vesicles was confirmed by immunoblotting using the antibody AC1.2, according to Hsu-Lin et al., 1984, supra; the incorporation of GPIIb-IIIa into vesicles was confirmed by the same technique using anti-GPIIb-IIIa. Vesicles were stored at 4°C under nitrogen in the dark. The vesicle preparation was diluted 1:5 with cells (1 x 10⁸/ml) suspended in RPMI 1640 with 1% fetal calf serum and 2% bovine serum albumin. After a 10 min incubation at 23°C the cells were sedimented at 16,000 x g for 15 sec. Cells were washed once with TBS and resuspended in the same buffer. Observation of fluorescence and phase contrast microscopy was performed using a Zeiss Axioscope microscope.

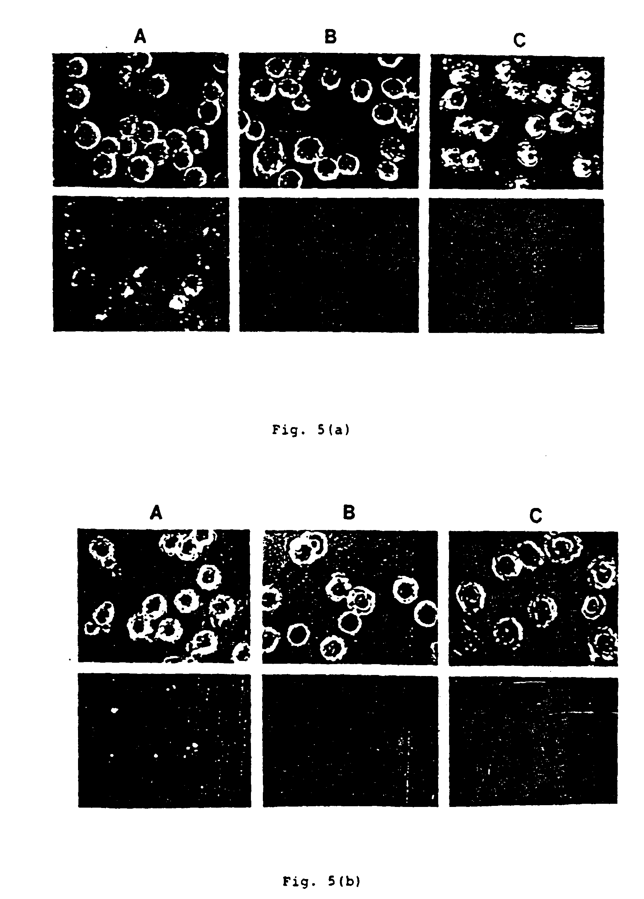

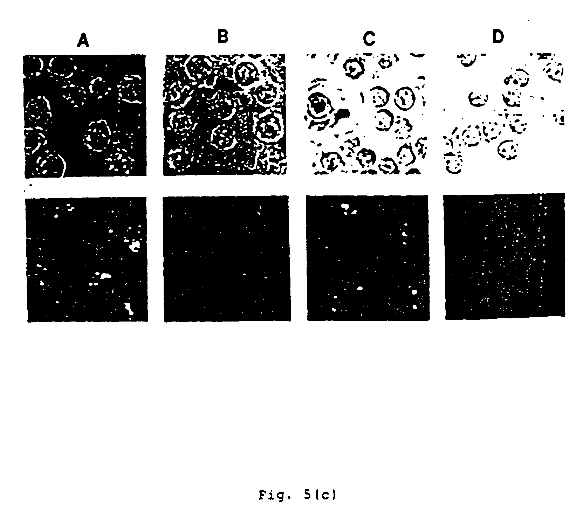

The interaction of the fluorescent vesicles with neutrophils, U937 cells and Jurkatt cells was studied by fluorescence microscopy and radioimmunoassay. The results are shown in Fig. 5. In each of Figs. 5a,b,c, the upper panels represent phase contrast micrographs and the lower panels fluorescence micrographs of the identical fields (Bar = 10 um). In Fig. 5a, fluorescent phospholipid vesicles composed of phosphatidylcholine and NBD-labeled phosphatidylcholine were incubated with U937 or Jurkatt cells; lane A represents U937 cells and PADGEM-containing phospholipid vesicles; lane B, Jurkatt cells and PADGEM-containing phospholipid vesicles; and lane C, U937 cells and phospholipid vesicles without PADGEM. Fig. 5a, lane A, shows that PADGEM-containing phosphilipid vesicles bind to the surface of U937 cells. Lanes B and C show that PADGEM-containing phospholipid vesicles did not bind to Jurkatt cells, and phospholipid vesicles lacking PADGEM did not bind to U937 cells, respectively. Phospholipid vesicles containing glycoprotein IIb-IIIa did not interact with U937 cells (data not shown).

in Fig. 5b, fluorescent phospholipid vesicles were incubated with neutrophils, as described above. The results in Fig. 5b show that when fluorescent vesicles were incubated with neutrophils, PADGEM-containing vesicles bind to neutrophils (lane A), and vesicles lacking PADGEM (lane B) or containing glycoprotein IIb-IIIa (lane C) did not interact with neutrophils.

The specificity of the PADGEM-mediated cellular interaction is demonstrated in Fig. 5c, in which anti-PADGEM antibodies are shown to cause inhibition of the interaction. In Fig. 5c, lane A, PADGEM-containing phospholipid vesicles are incubated with and bind to U937 cells; in lane B, this cellular interaction is inhibited in the presence of anti-PADGEM antibodies. In Fig. 5c, lane C, PADGEM-containing phospholipid vesicles are incubated with and bind to neutrophils; and in lane D, anti-PADGEM antibodies inhibit this interaction. These results demonstrate that PADGEM-vesicle binding to U937 cells and to neutrophils is specifically mediated by PADGEM.

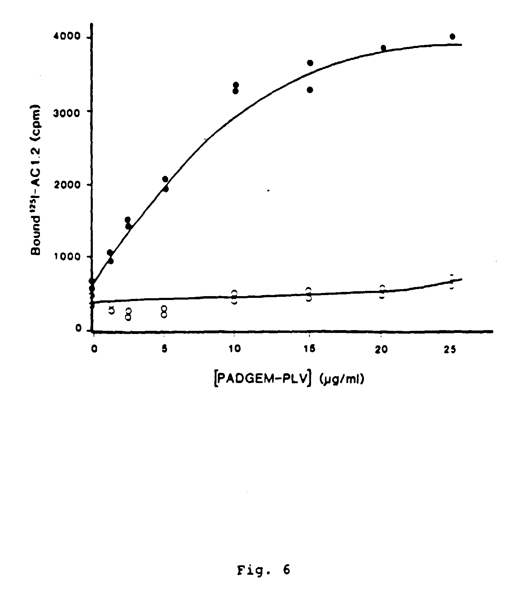

The interaction of PADGEM-containing phospholipid containing vesicles with U937 cells was quantitated using ¹²⁵I-labeled AC1.2 antibody, as follows. The vesicle preparation was diluted 1:1 in RPMI 1640 containing 1% fetal calf serum and 2% bovine serum albumin. After pre-incubation with a fixed amount of ¹²⁵I-labeled AC1.2 antibody, which does not inhibit activated platelet-U937 cell interaction, for 45 min, at 37°C, antibody-PADGEM/vesicle complex was added to the cell suspension at indicated concentrations and incubated for 30 min. at 37°C. The unbound vesicles were separated from cell-bound vesicles by centrifugation at 10,000 x g for 5 min through a layer of oil (n-butyl phthalate (Aldrich Chem., Milwaukee, WI):Apiezon oil 93:7 v/v), and the cellular sediment assayed for ¹²⁵I. The concentration of PADGEM exposed on vesicles was estimated based upon random orientation of PADGEM on the inner and outer aspect of the phospholipid bilayer. In Fig. 6, the concentration given on the x-axis (PADGEM-PLV) is the total contentration of PADGEM; closed circles represent U937 cells and open circles represent Jurkatt cells. The results in Fig. 6 show that the interaction of PADGEM-containing vesicles with U937 cells is specific and saturable; minimal binding of vesicles was noted with Jurkatt cells employed as a control.

### Screening of PADGEM Protein Fragments

Various PADGEM protein fragments may be generated by proteolysis of purified PADGEM protein, e.g., by trypsin digestion, separated according to conventional techniques, and individually screened in the HL60/platelet adhesion assay described below. (HL60 cells are publicly available cells which exhibit binding properties of monocytes.) Alternatively, peptides of a chosen length and corresponding in amino acid sequence to a region of a natural PADGEM protein fragment may be synthesized according to conventional techniques, and then screened.

Fragments of PADGEM protein can be screened for competitive inhibition of PADGEM-promoted cellular adhesion by adding each fragment preparation to the well of a plate coated with a uniform monolayer of activated platelets and to which radioactively-labeled HL60 cells are also added. Those fragments which inhibit HL60 cell binding to the plate act as inhibitors of cell-cell binding.

### Preparation of Platelet Monolayers

Platelets may be prepared and activated as follows. Forty-five ml of whole blood are drawn in a 50 ml syringe containing 5 ml of Ware's anticoagulant (3 parts 0.1 M Na Citrate + 2 parts 0.1 M citric acid) containing 10 mM acetylsalicylic acid (1 mM final concentration). Platelet-rich plasma is separated from red blood cells and leukocytes by spinning the blood at 1100 rpm in the International Centrifuge (source) and aspirated into a clean plastic tube. 7 ml of platelet-rich plasma (PRP) is applied to a 50 ml Sepharose 2B column (15 cm x 2 cm column) which has been washed extensively with distilled water (500 ml) followed by HEPES buffer (500 ml). All PRP is allowed to enter the gel, and the buffer is then restarted. Once the flow-through buffer becomes turbid, platelet collection is begun in one ml fractions. The first ml of gel filtered platelets is discarded and collection is continued until clearing of the buffer occurs. The platelets are collected by running the turbid elution buffer down the sides of plastic collection tubes so as to minimize agitation. The total volume and concentration of platelets are recorded and the gel filtered platelets are diluted to a final concentration of 10⁸ cells/ml in HEPES buffer. EDTA is then added to a final concentation of 2.5 mM. If resting platelets are desired then no further preparation is necessary.

Activated platelets are prepared by adding thrombin to the resting gel filtered platelets to a final concentration of 0.15 units/ml and incubating at room temperature for 20 minutes. Care is taken not to agitate platelets during incubation.

Uniformly coated platelet monolayer plates are prepared as follows. Prior to preparation of platelets, 96-well tissue culture plates (Becton Dickinson Co., Cockeysville, MD) are coated for 1 hour with poly-L-lysine solution (200 ug/ml in phosphate buffered saline) (100 µl/well); the wells are then aspirated and air dried before use. Once dried, plates can be stored at -80° C if desired. To each coated well, 100 µl 4% paraformaldehyde fixative is added and plates are incubated at 37° C. 4% paraformaldehyde fixative is prepared by stirring 4 gm paraformaldehyde into 60° C 50 ml dH₂O, then adding 1-3 drops 1 N NaOH until the solution clears. After the paraformaldehyde mixture is cooled, it is filtered with a 0.22 µ filter and then mixed 1:1 with 0.2 M phosphate buffer pH 7.2. Once the temperature of the plates is equilibrated at 37° C, 10⁷ platelets (100 µl suspension) are added to each well and the plates are spun at 2000 g for 15 min. at 4° C. Plates are then further incubated at 4° C for an additional 45 min. and then washed three times with Tris Buffered Saline (0.02 M Tris/Cl, 0.14 M NaCl, pH 7.4) (TBS)/20 mM ammonium chloride, and then a fourth time with TBS alone. Plates can then be stored at 4° C without any noticeable interruption of the platelets monolayer.

### Mononuclear Cell Labelling

HL60 cells or other mononuclear cells are pelleted at 200 g for 5 min. and suspended gently in Hank's Balanced Salt Solution (HBSS) plus 2 mM CaCl₂, 2 mM MgCl₂ with 1% fetal calf serum (assay buffer) at a final concentration of 10⁷ cells per ml. Indium -111 (Amersham Clinical, Arlington Heights, IL) (1 mCi/ml) is added at 1 µci/10⁶ cells and cells incubated at room temperature for 15 min. Cells are spun at 200 g, washed twice with assay buffer and suspended in the same solution at a concentration of 10⁵ cells per 100 µl. Labelling efficiency is measured by counting the total counts in 100 µl of HL60 suspension and in 100 µl of supernatant obtained after centrifugation. Pecent labelling is calculated by the following formula: total counts - supernatant counts/ total counts. Typically, 90-95% of counts are associated with cells.

### Binding Assay

Storage buffer is removed from the platelet coated wells and the wells are washed once with assay buffer and emptied. The PADGEM protein fragment preparations are each dissolved in assay buffer and added to appropriate wells in the desired concentration. It will be desirable to test each fragment preparation over a range of concentrations. Wells are then filled to a volume of 190 µl with assay buffer; control wells are filled with assay buffer. All samples and controls are tested in multiples of 3-6. HL60 or other labeled cells are added (10⁵/well) and plates are spun for 5 min. at 100g. Plates are incubated at room temperature for a total of 20 min. and then are sealed with cellulose acetate plate sealer (Dynatech, Alexandria, VA) and centrifuged inverted at 5 g for 10 min. The fluid and nonadherent HL60 cells are carefully emptied while the plates are inverted. The sides of the wells are dried with cotton swabs with care so as not to disturb the platelet monolayer and adherent cells.

### Measurement of Binding and Percent Inhibition

Before solubilizing the contents of each well, the well is inspected visually with an inverted microscope for the presence of binding and a visual estimate is made of percent inhibition of binding. After all wells are inspected, cells are lysed with 100 µl/well of 2% SDS for 15 min. and contents of the wells are transferred to tubes for counting in a gamma counter. Percent inhibition is determined by comparison of counts retained in experimental wells relative to control wells in each experiment. A high value of counts retained in a well indicates that a high percentage of ¹¹¹I-labeled HL60 cells bound to the platelet monolayer, i.e., the cells were not competitively inhibited from binding to platelets.

### Screening of Cell-Cell Binding Inhibitor Carbohydrates

As mentioned above, competitive inhibition of PADGEM-mediated cell-cell binding can be achieved not only by use of PADGEM protein fragments, but also by use of carbohydrates capable of selectively binding to PADGEM, i.e., carbohydrates which mimic or are identical in their binding characteristics the PADGEM-specific ligand present on certain monocytes and neutrophils. Candidate carbohydrates are screened in the method described above using HL60 cells and platelets.

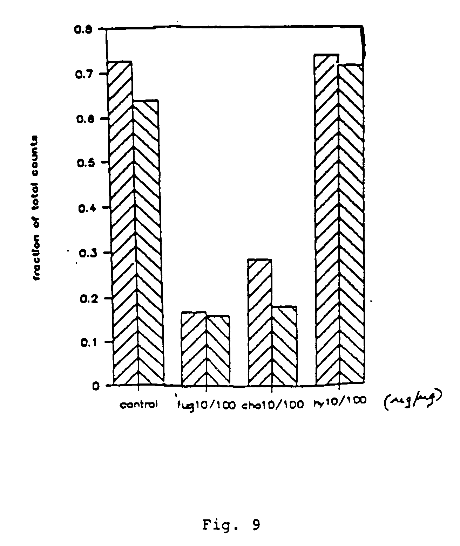

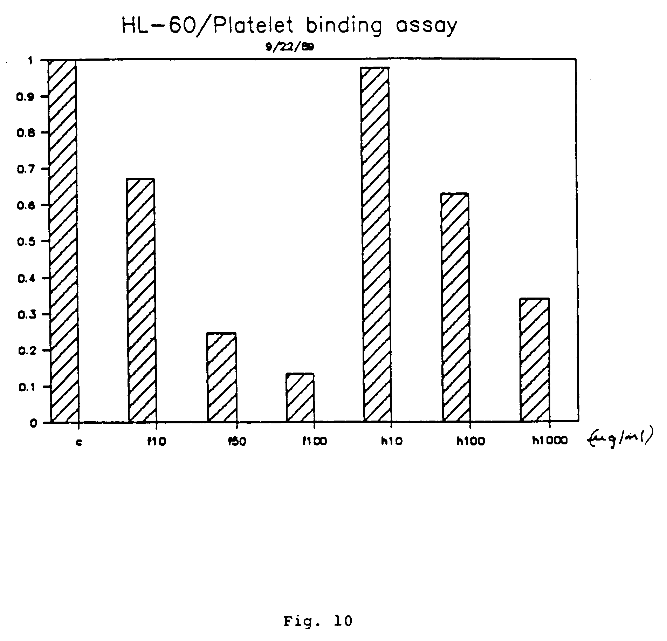

Referring to Figs. 9 and 10, four commercially available polymeric carbohydrates were tested for their ability to inhibit HL60-platelet binding. Those carbohydrates were fucoidin, chondroitin sulfate, heparin, and hyaluronic acid. One other carbohydrate compound, the simple sugar galactose sulfate, was also tested (results not shown in Figs.). As is shown in Figs. 9 and 10, fucoidin, chondroitin sulfate, and heparin, all effectively inhibited binding. Hyaluronic acid, which is not sulfated, was not effective, nor was galactose sulfate or mannose-6-sulfate.

### Production of PADGEM Protein Fragments

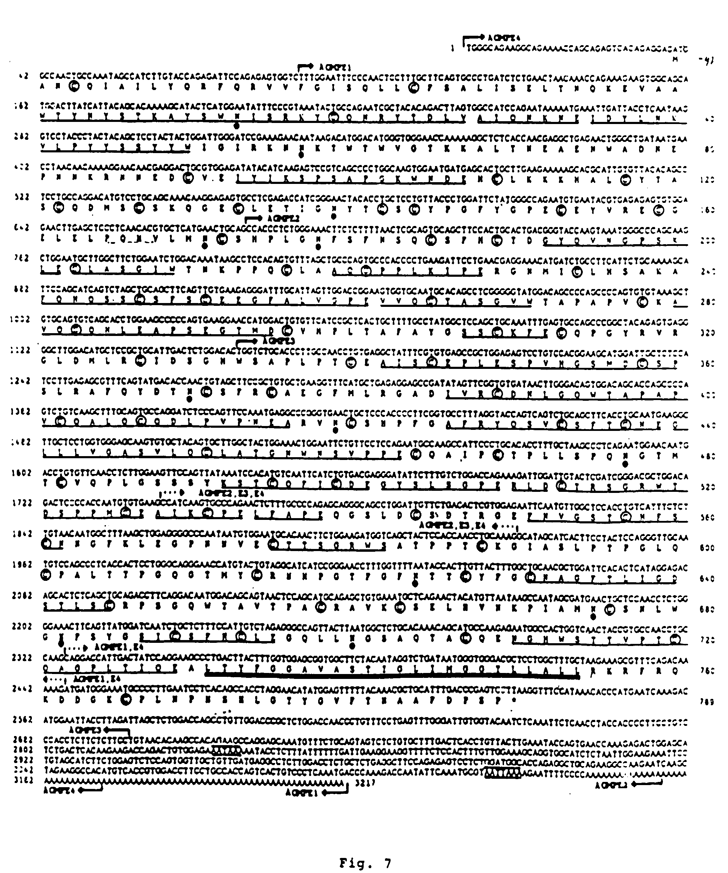

Fig. 7 shows the DNA and corresponding protein sequence for the PADGEM gene and protein, as described in Johnston et al., supra. The nucleotide sequence shown in Fig. 7 was compiled by Johnston et al. from four overlapping cDNA clones λGMPE1-λGMPE4 and the relative positions of these cDNAS are shown by the solid arrows. Dotted arrows indicate regions found in some clones but deleted in others. The numbering of nucleotides in the figure arbitrarily starts at the first base following the adapter oligonucleotide sequence of the most 5' clone. The translated amino acid sequence of the open reading frame within the gene is given in the single-letter code. The initiating methionine was assigned to the first in-frame ATG sequence that agreed with the consensus sequence for initiation of translation. The stop codon is shown by the asterisk. The thin underlines show the matching positions of amino acid sequences determined from the N-terminus and from 26 peptides of platelet PADGEM. The signal peptide corresponds to positions -41 to -1. The putative transmembrane domain is heavily underlined. The cysteine residues are shown as open circles, and the potential aspargine-linked glycosylation sites (NxS/T) are shown by the filled circles. Two potential polyadenylation signals in the 3' untranslated region are underlined and overlined.

The PADGEM gene may be obtained as described in Johnston et al., supra, and digested with restriction enzymes to generate a desired DNA fragment; the fragment may then be cloned, expressed, and the resulting protein fragment purified, all according to conventional techniques well-known in the art; e.g., see Maniatis et al., Eds., Molecular Cloning, A Laboratory Manual, Cold Spring Harbor, NY, 1982, and Pouwels et al., Eds., Cloning Vectors, Elsevier, Amsterdam, 1985, 1987. Alternatively, the nucleotide sequence shown in Fig. 7 may be used to generate synthetic DNA molecules encoding either a desired region of the PADGEM protein or the complete protein, and the synthetic DNA may then be cloned, expressed, and the protein or protein fragment purified according to conventional techniques. If the entire protein is produced in this way, it may be digested with proteolytic enzymes to generate the desired fragment. Finally, the deduced amino acid sequence of PADGEM, as shown in Fig. 7, may be used to generate a synthetic peptide.

### Use

A soluble PADGEM protein fragment or carbohydrate may be administered to a human in one of the traditional modes, (e.g., orally, intravenously, parenterally or transdermally in a sustained release formulation using a biodegradable biocompatible polymer) admixed with an appropriate carrier or diluent, or using micelles, gels, or liposomes.

The protein fragment or carbohydrate can be administered to a human patient in a dosage of 0.5 µg/kg/day to 5 µg/kg/day.

## Claims

1. A method of inhibiting, in vitro, the binding of a first cell bearing PADGEM (platelet activation dependent granule - external membrane protein) to a second cell bearing a PADGEM-specific ligand, comprising contacting said sample with an inhibiting substance which binds either to PADGEM or to said PADGEM-specific ligand to inhibit the binding of said first cell to said second cell.

2. A method according to Claim 1, wherein said first cell is an activated platelet.

3. A method according to Claim 1, wherein said first cell is an endothelial cell.

4. A method according to Claim 1, wherein said second cell is a monocyte.

5. A method according to Claim 1, wherein said second cell is a neutrophil.

6. A method according to Claim 1, wherein said first cell is a stimulated endothelial cell and said second cell is a monocyte or neutrophil.

7. A method according to Claim 1, wherein said first cell is an activated platelet and said second cell is a monocyte or neutrophil.

8. A method according to Claim 1, wherein said inhibiting substance comprises a soluble protein fragment capable of mimicking a PADGEM ligand-specific binding site of PADGEM.

9. A method according to Claim 1, wherein said inhibiting substance comprises a soluble carbohydrate capable of binding to PADGEM on said first cell.

10. A method according to Claim 1, wherein said inhibitory substance is an antibody to PADGEM.

11. A soluble protein fragment capable of mimicking a site of PADGEM which binds to a PADGEM-specific ligand on a leukocyte.

12. A fragment according to Claim 11, wherein said leukocyte is a monocyte or a neutrophil.

13. A fragment according to Claim 11, excluding the transmembrane region of PADGEM, or including only a portion of said transmembrane region small enough not to prevent solubilization of said fragment.

14. A fragment according to Claim 13, including the lectin domain of PADGEM.

15. A fragment according to Claim 13, including a C3b-C4b regulatory protein repeat domain of PADGEM.

16. A fragment according to Claim 13, said fragment being at least 90% homologous with a region of PADGEM.

17. A fragment according to Claim 13, said fragment containing at least four amino acids.

18. A fragment according to Claim 13, said fragment containing at least ten amino acids.

19. A therapeutic composition comprising two or more different soluble fragments as defined in Claim 11.

20. An expression vector comprising a DNA sequence encoding a soluble fragment according to Claim 11.

21. A cell comprising an expression vector according to Claim 20.

22. A method of making a soluble PADGEM fragment comprising culturing a cell according to Claim 21 and isolating said soluble fragment therefrom.

23. Use of a composition comprising an inhibiting substance which binds to PADGEM for the manufacture of a medicament for the inhibition of the binding of a first cell bearing PADGEM to a second cell bearing a PADGEM specific ligand.

24. Use of a composition comprising an inhibiting substance which binds to PADGEM for the manufacture of medicament as defined in claim 23 wherein said first cell is an activated platelet or an endothelial cell.

25. Use of a composition comprising an inhibiting substance which binds to PADGEM for the manufacture of medicament as defined in claim 23 wherein said inhibiting substance comprises a soluble carbohydrate capable of binding to PADGEM on said first cell.

26. Use of a composition comprising an inhibiting substance which binds to PADGEM for the manufacture of a medicament as defined in claim 23 wherein said inhibiting substance is an antibody to PADGEM.

27. Use of a composition comprising an inhibiting substance which binds to PADGEM for the manufacture of a medicament as defined in claim 23 wherein said first cell is a stimulated endothelial cell, said second cell is a monocyte or neutrophil and said inhibition results in an inhibition of atherosclerosis.

28. Use of a composition comprising an inhibiting substance which binds to PADGEM for the manufacture of a medicament as defined in claim 23 wherein said first cell is an activated platelet and said second cell is a monocyte or neutrophil and said inhibition results in an inhibition of clotting or inflammation.

29. Use of a composition comprising an inhibiting substance which binds to a PADGEM specific ligand for the manufacture of a medicament for the inhibition of the binding of a first cell bearing PADGEM to a second cell bearing said PADGEM specific ligand.

30. Use of a composition comprising an inhibiting substance which binds to a PADGEM specific ligand for the manufacture of a medicament as defined in claim 29 wherein said second cell is a monocyte or a neutrophil.

31. Use of a composition comprising an inhibiting substance which binds to a PADGEM specific ligand for the manufacture of a medicament as defined in claim 29, wherein said inhibiting substance comprises a soluble protein fragment capable of mimicking a PADGEM ligand specific binding site of PADGEM.

32. Use of a composition comprising an inhibiting substance which binds to a PADGEM specific ligand for the manufacture of a medicament as defined in claim 29 wherein said first cell is a stimulated endothelial cell and said second cell is a neutrophil or monocyte and said inhibition results in an inhibition of atherosclerosis.

33. Use of a composition comprising an inhibiting substance which binds to PADGEM specific ligand for the manufacture of a medicament as defined in claim 29 wherein said first cell is an activated platelet, said second cell is a neutrophil or a monocyte and said inhibition results in an inhibition of clotting or inflammation.

## Patentansprüche

1. Verfahren zur in-vitro Inhibierung der Bindung einer ersten Zelle, die PADGEM (platelet activation dependent granule-external membrane protein, Blutplättchenaktivierung abhängiges externes Granulozytenmembranprotein) trägt, an eine zweite Zelle, die einen PADGEM-spezifischen Liganden trägt, wobei man die Probe mit einer Inhibierungssubstanz in Kontakt bringt, die entweder an PADGEM oder an den PADGEM-spezifischen Liganden bindet, um die Bindung der ersten Zelle an die zweite Zelle zu inhibieren.

2. Verfahren nach Anspruch 1, wobei die erste Zelle ein aktiviertes Blutplättchen ist.

3. Verfahren nach Anspruch 1, wobei die erste Zelle eine Endothelzelle ist.

4. Verfahren nach Anspruch 1, wobei die zweite Zelle ein Monozyt ist.

5. Verfahren nach Anspruch 1, wobei die zweite Zelle ein neutrophiler Leukozyt ist.

6. Verfahren nach Anspruch 1, wobei die erste Zelle eine stimulierte Endothelzelle und die zweite Zelle ein Monozyt oder ein neutrophiler Leukozyt ist.

7. Verfahren nach Anspruch 1, wobei die erste Zelle ein aktiviertes Blutplättchen und die zweite Zelle ein Monozyt oder ein neutrophiler Leukozyt ist.

8. Verfahren nach Anspruch 1, wobei die Inhibierungssubstanz ein lösliches Proteinfragment umfaßt, das in der Lage ist, eine PADGEM-Ligand-spezifische Bindungsstelle von PADGEM nachzuahmen.

9. Verfahren nach Anspruch 1, wobei die Inhibierungssubstanz ein lösliches Kohlenhydrat umfaßt, das in der Lage ist, an PADGEM der ersten Zelle zu binden.

10. Verfahren nach Anspruch 1, wobei die Inhibierungssubstanz ein Antikörper gegen PADGEM ist.

11. Lösliches Proteinfragment, das in der Lage ist eine Stelle von PADGEM nachzuahmen, die an einen PADGEM-spezifischen Liganden auf einem Leukozyten bindet.

12. Fragment nach Anspruch 11, worin der Leukozyt ein Monozyt oder ein neutrophiler Leukozyt ist.

13. Fragment nach Anspruch 11, ausschließlich der transmembranen Region von PADGEM oder einschließlich nur eines Teils der transmembranen Region, der klein genug ist, die Solubilisierung des Fragments nicht zu verhindern.

14. Fragment nach Anspruch 13, einschließlich der Lectindomäne von PADGEM.

15. Fragment nach Anspruch 13, einschließlich einer repetitiven C3b-C4b-Regulatorproteindomäne von PADGEM.

16. Fragment nach Anspruch 13, worin das Fragment zu mindestens 90 % homolog mit einer Region von PADGEM ist.

17. Fragment nach Anspruch 13, worin das Fragment mindestens vier Aminosäuren umfaßt.

18. Fragment nach Anspruch 13, worin das Fragment mindestens zehn Aminosäuren umfaßt.

19. Therapeutische Zusammensetzung, umfassend zwei oder mehrere unterschiedliche lösliche Fragmente nach Anspruch 11.

20. Expressionsvektor, umfassend eine DNA-Sequenz, die für ein lösliches Fragment nach Anspruch 11 codiert.

21. Zelle, umfassend einen Expressionsvektor nach Anspruch 20.

22. Verfahren zur Herstellung eines löslichen PADGEM-Fragments, wobei man eine Zelle nach Anspruch 21 züchtet und daraus das lösliche Fragment isoliert.

23. Verwendung einer Zusammensetzung, umfassend eine Inhibierungssubstanz, die an PADGEM bindet, zur Herstellung eines Arzneimittels zur Inhibierung der Bindung einer ersten Zelle, die PADGEM trägt, an eine zweite Zelle, die einen PADGEM-spezifischen Liganden trägt.

24. Verwendung einer Zusammensetzung, umfassend eine Inhibierungssubstanz, die an PADGEM bindet, zur Herstellung eines Arzneimittels nach Anspruch 23, worin die erste Zelle ein aktiviertes Blutplättchen oder eine Endothelzelle ist.

25. Verwendung einer Zusammensetzung, umfassend eine Inhibierungssubstanz, die an PADGEM bindet, zur Herstellung eines Arzneimittels nach Anspruch 23, worin die Inhibierungssubstanz ein lösliches Kohlenhydrat umfaßt, das in der Lage ist, an PADGEM der ersten Zelle zu binden.

26. Verwendung einer Zusammensetzung, umfassend eine Inhibierungssubstanz, die an PADGEM bindet, zur Herstellung eines Arzneimittels nach Anspruch 23, worin die Inhibierungssubstanz ein Antikörper gegen PADGEM ist.

27. Verwendung einer Zusammensetzung, umfassend eine Inhibierungssubstanz, die an PADGEM bindet, zur Herstellung eines Arzneimittels nach Anspruch 23, worin die erste Zelle eine stimulierte Endothelzelle, die zweite Zelle ein Monozyt oder ein neutrophiler Leukozyt ist, und die Inhibierung zu einer Hemmung der Atherosclerose führt.

28. Verwendung einer Zusammensetzung, umfassend eine Inhibierungssubstanz, die an PADGEM bindet, zur Herstellung eines Arzneimittels nach Anspruch 23, worin die erste Zelle ein aktiviertes Blutplättchen und die zweite Zelle ein Monozyt oder ein neutrophiler Leukozyt ist, und die Inhibierung zu einer Hemmung der Gerinnung oder Entzündung führt.

29. Verwendung einer Zusammensetzung, umfassend eine Inhibierungssubstanz, die an einen PADGEM-spezifischen Liganden bindet, zur Herstellung eines Arzneimittels zur Inhibierung der Bindung einer ersten Zelle, die PADGEM trägt, an eine zweite Zelle, die den PADGEM-spezifischen Liganden trägt.

30. Verwendung einer Zusammensetzung, umfassend eine Inhibierungssubstanz, die an einen PADGEM-spezifischen Liganden bindet, zur Herstellung eines Arzneimittels nach Anspruch 29, worin die zweite Zelle ein Monozyt oder ein neutrophiler Leukozyt ist.

31. Verwendung einer Zusammensetzung, umfassend eine Inhibierungssubstanz, die an einen PADGEM-spezifischen Liganden bindet, zur Herstellung eines Arzneimittels nach Anspruch 29, worin die Inhibierungssubstanz ein lösliches Proteinfragment umfaßt, das in der Lage ist, eine PADGEM-Ligand-spezifische Bindungsstelle von PADGEM nachzuahmen.

32. Verwendung einer Zusammensetzung, umfassend eine Inhibierungssubstanz, die an einen PADGEM-spezifischen Liganden bindet, zur Herstellung eines Arzneimittels nach Anspruch 29, worin die erste Zelle eine stimulierte Endothelzelle und die zweite Zelle ein neutrophiler Leukozyt oder Monozyt ist, und die Inhibierung zu einer Hemmung der Atherosclerose führt.

33. Verwendung einer Zusammensetzung, umfassend eine Inhibierungssubstanz, die an einen PADGEM-spezifischen Liganden bindet, zur Herstellung eines Arzneimittels nach Anspruch 29, worin die erste Zelle ein aktiviertes Blutplättchen, die zweite Zelle ein neutrophiler Leukozyt oder Monozyt ist, und die Inhibierung zu einer Hemmung der Gerinnung oder Entzündung führt.

## Revendications

1. Procédé d'inhibition in vitro de la liaison d'une première cellule portant PADGEM (protéine de membrane externe-granulation dépendante de l'activation plaquettaire) à une deuxième cellule portant un ligand spécifique de PADGEM, comprenant la mise en contact dudit échantillon avec une substance inhibitrice qui se combine à PADGEM ou audit ligand spécifique de PADGEM pour inhiber la liaison de ladite première cellule à ladite deuxième cellule.

2. Procédé selon la revendication 1, dans lequel ladite première cellule est une plaquette activée.

3. Procédé selon la revendication 1, dans lequel ladite première cellule est une cellule endothéliale.

4. Procédé selon la revendication 1, dans lequel ladite deuxième cellule est un monocyte.

5. Procédé selon la revendication 1, dans lequel ladite deuxième cellule est un neutrophile.

6. Procédé selon la revendication 1, dans lequel ladite première cellule est une cellule endothéliale stimulée et ladite seconde cellule est un monocyte ou un neutrophile.

7. Procédé selon la revendication 1, dans lequel ladite première cellule est une plaquette activée et ladite deuxième cellule est un monocyte ou un neutrophile.

8. Procédé selon la revendication 1, dans lequel ladite substance inhibitrice comprend un fragment protéique soluble capable de simuler un site de fixation de PADGEM spécifique du ligand de PADGEM.

9. Procédé selon la revendication 1, dans lequel ladite substance inhibitrice comprend un hydrate de carbone soluble capable de se combiner à PADGEM sur ladite première cellule.

10. Procédé selon la revendication 1, dans lequel ladite substance inhibitrice est un anticorps dirigé contre PADGEM.

11. Fragment protéique soluble capable de simuler un site de PADGEM qui se combine à un ligand spécifique de PADGEM sur un leucocyte.

12. Fragment selon la revendication 11, dans lequel ledit leucocyte est un monocyte ou un neutrophile.

13. Fragment selon la revendication 11, excluant la région transmembranaire de PADGEM ou comprenant uniquement une partie de ladite région transmembranaire suffisamment petite pour ne pas empêcher la solubilisation dudit fragment.

14. Fragment selon la revendication 13, comprenant le domaine de lectine de PADGEM.

15. Fragment selon la revendication 13, comprenant une séquence répétée protéique régulatrice C3b-C4b de PADGEM.

16. Fragment selon la revendication 13, ledit fragment étant homologue à au moins 90 % avec une région de PADGEM.

17. Fragment selon la revendication 13, ledit fragment contenant au moins 4 acides aminés.

18. Fragment selon la revendication 13, ledit fragment contenant au moins 10 acides aminés.

19. Composition thérapeutique comprenant deux fragments solubles différents ou plus tels que définis à la revendication 11.

20. Vecteur d'expression comprenant une séquence d'ADN codant un fragment soluble selon la revendication 11.

21. Cellule comprenant un vecteur d'expression selon la revendication 20.

22. Procédé de préparation d'un fragment soluble de PADGEM, comprenant la culture d'une cellule selon la revendication 21 et l'isolement dudit fragment soluble de cette dernière.

23. Utilisation d'une composition comprenant une substance inhibitrice qui se combine à PADGEM en vue de la préparation d'un médicament destiné à l'inhibition de la liaison d'une première cellule portant PADGEM à une deuxième cellule portant un ligand spécifique de PADGEM.

24. Utilisation d'une composition comprenant une substance inhibitrice qui se combine à PADGEM en vue de la préparation d'un médicament selon la revendication 23, où ladite première cellule est une plaquette activée ou une cellule endothéliale.

25. Utilisation d'une composition comprenant une substance inhibitrice qui se combine à PADGEM en vue de la préparation d'un médicament selon la revendication 23, où ladite substance inhibitrice comprend un hydrate de carbone soluble capable de se combiner à PADGEM sur ladite première cellule.

26. Utilisation d'une composition comprenant une substance inhibitrice qui se combine à PADGEM en vue de la préparation d'un médicament selon la revendication 23, où ladite substance inhibitrice est un anticorps dirigé contre PADGEM.

27. Utilisation d'une composition comprenant une substance inhibitrice qui se combine à PADGEM en vue de la préparation d'un médicament selon la revendication 23, où ladite première cellule est une cellule endothéliale stimulée, ladite deuxième cellule est un monocyte ou un neutrophile et ladite inhibition aboutit à une inhibition de l'athérosclérose.

28. Utilisation d'une composition comprenant une substance inhibitrice qui se combine à PADGEM en vue de la préparation d'un médicament selon la revendication 23, où ladite première cellule est une plaquette activée et ladite deuxième cellule est un monocyte ou un neutrophile et ladite inhibition aboutit à l'inhibition de la coagulation ou de l'inflammation.

29. Utilisation d'une composition comprenant une substance inhibitrice qui se combine à un ligand spécifique de PADGEM en vue de la préparation d'un médicament destiné à l'inhibition de la liaison d'une première cellule portant PADGEM à une deuxième cellule portant ledit ligand spécifique de PADGEM.

30. Utilisation d'une composition comprenant une substance inhibitrice qui se combine à un ligand spécifique de PADGEM en vue de la préparation d'un médicament selon la revendication 29, où ladite deuxième cellule est un monocyte ou un neutrophile.

31. Utilisation d'une composition comprenant une substance inhibitrice qui se combine à un ligand spécifique de PADGEM en vue de la préparation d'un médicament selon la revendication 29, où ladite substance inhibitrice comprend un fragment protéique soluble capable de simuler un site de fixation de PADGEM spécifique du ligand de PADGEM.

32. Utilisation d'une composition comprenant une substance inhibitrice qui se combine à un ligand spécifique de PADGEM en vue de la préparation d'un médicament selon la revendication 29, où ladite première cellule est une cellule endothéliale stimulée et ladite deuxième cellule est un neutrophile ou un monocyte et ladite inhibition aboutit à une inhibition de l'athérosclérose.

33. Utilisation d'une composition comprenant une substance inhibitrice qui se combine à un ligand spécifique de PADGEM en vue de la préparation d'un médicament selon la revendication 29, où ladite première cellule est une plaquette activée, ladite deuxième cellule est un neutrophile ou un monocyte et ladite inhibition aboutit à une inhibition de la coagulation ou de l'inflammation.
